# EUROPEAN PATENT APPLICATION

(11) **EP 3 289 973 A1**
(43) Date of publication of application: **07.03.2018**
(21) Application number: 16187294.0
(22) Date of filing: 05.09.2016
(51) Int. Cl.: A61B 5/055, G01R 33/20

(54) **PATIENT POSITIONING APPARATUS**

(71) Applicant: Medical Intelligence Medizintechnik GmbH, 86830 Schwabmünchen (DE)
(72) Inventor: LIU, Rui, Augsburg 86153 (DE); WEBER, Florian, Freising 85354 (DE); XU, Gavin Ziyuan, Crawley, West Sussex, RH10 1AU (GB)
(74) Representative: Finnegan Europe LLP

(57) **Abstract**

An apparatus for positioning a patient for medical imaging comprises a support system (10), a control unit (20) and a filter unit (30). The support system (10) comprises a patient support device (12) and an actuator (14) configured to move the patient support device. The support system (10) is located inside an electromagnetically-shielded enclosure (2). The control unit (20) is configured to adjust the position of the patient support device (12) by communicating with the support system (10) via one or more signal lines (40). The control unit (20) is located outside the electromagnetically-shielded enclosure (2). The filter unit (30) is configured to filter noise from the signal lines (40).

## Description

The present disclosure generally relates to an apparatus for positioning a patient for medical imaging. The present disclosure also relates to a filter unit for filtering noise at the scan frequency of a medical imaging device.

Medical imaging is commonly performed to assist in the diagnosis and/or treatment of various medical conditions. Magnetic resonance imaging (MRI) is an example of a medical imaging technology that is often performed during the diagnosis and treatment of tumours.

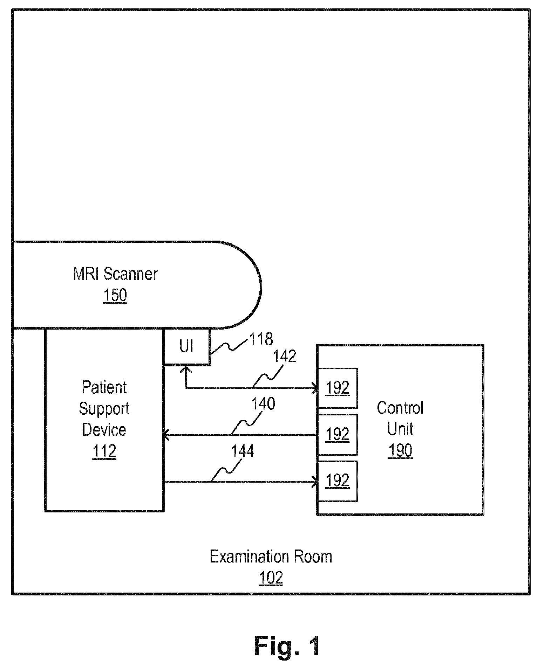

Figure 1 is a schematic diagram of a conventional MRI system. The MRI system comprises an MRI scanner 150, a patient support device 112, and a control unit 190 for the patient support device 112, all of which are located within an examination room 102. The MRI scanner 150 has a bore within which the body of a patient is situated during an MRI process. The patient support device 112 supports the body of a patient during the MRI process. The patient support device 112 is movable, so as to allow the patient to be moved into and out of the bore of the MRI scanner 150. Movement of the patient support device 112 is effected by an electric motor, which receives a control signal from the control unit 190 via a first cable 140. A user interface (UI) 118 is electrically connected to the control unit 190 via a second cable 142. The control unit 190 controls the electric motor in response to a user input received via the user interface 118, thereby causing movement of the support device 112. The control unit 190 also receives a feedback signal, which indicates the position of the patient support device 112, via a third cable 144.

The presence of electromagnetic noise in the vicinity of the MRI scanner 150 can adversely affect the quality of the medical images produced. Thus, a conventional MRI system, such as that shown in Figure 1, has several features to reduce the level of electromagnetic noise in the vicinity of the MRI scanner 150. First, the examination room 102 is electromagnetically shielded. Second, the control unit 190 is contained within an electromagnetically-shielded housing. Third, the control unit 190 has a feedthrough filter 192 connected to each of the cables 140, 142, 144. The feedthrough filter 192 aims to prevent noise generated by electrical components in the control unit 190 being transmitted into the examination room 102 via the cables 140, 142, 144. Finally, the control unit 190 is implemented with specially-designed analogue and digital circuits. These circuits operate at a relatively low frequency and generate less radio frequency (RF) electromagnetic noise than microprocessor-based control systems, and also operate at a lower frequency than the scan frequency of the MRI scanner 150. As a result, noise generated by the circuits can be easily filtered by the feedthough filters 192. The low noise level of these circuits satisfies the desire to minimise RF noise in the vicinity of the MRI scanner 150 and, therefore, the design of the control unit 190 has changed little since the 1980s.

However, the design of the conventional control unit 190 also limits the accuracy and precision with which the patient support device 112 can be moved. For example, the low frequency of the specially-designed circuits prevents the feedback signal received by the control unit 190 via the third cable 144 being sampled at a sufficiently high frequency to achieve accurate and precise positioning of the patient support device 112. The frequency of the circuits in the control unit 190 cannot simply be increased, however, since doing so could increase the level of RF noise in the vicinity of the MRI scanner 150 to a level that would adversely affect the quality of MRI images.

### SUMMARY

The present disclosure aims to overcome or mitigate the above-mentioned disadvantages of conventional apparatuses for positioning a patient for medical imaging. For example, the present disclosure can allow more accurate and precise control systems to be used for controlling the position of a patient support device, without increasing the level of electromagnetic noise in the vicinity of a medical imaging device.

A first aspect of the present disclosure provides an apparatus for positioning a patient for medical imaging, the apparatus comprising: a support system comprising a patient support device and an actuator configured to move the patient support device, wherein the support system is located inside an electromagnetically-shielded enclosure; a control unit configured to adjust the position of the patient support device by communicating with the support system via one or more signal lines, wherein the control unit is located outside the electromagnetically-shielded enclosure; and a filter unit configured to filter noise from the signal lines.

The filter unit may be located in a first aperture in a wall of the electromagnetically-shielded enclosure. The filter unit may further include an electrically-conductive housing, the housing being electrically connected to the wall. The filter unit may comprise a filter circuit having a stopband, wherein the filter circuit is configured such that a scan frequency of a medical imaging device is within the stopband. The filter circuit may be a passive filter including a ladder network of one or more inductors and one or more capacitors.

The control unit may be configured to provide a signal to the actuator via a first signal line of the one or more signal lines, and the actuator may be configured to move the patient support device in response to receiving the signal. The support system may further include a sensor configured to output a signal indicative of a position or movement of the patient support device, and the control unit may be further configured to receive the signal indicative of the position or movement of the patient support device via a second signal line of the one or more signal lines. The support system may further include a user interface configured to: receive a user input indicative of a desired position or a desired movement of the patient support device; and output a signal indicative of the desired position or desired movement of the patient support device to the control unit via a third signal line of the one or more signal lines.

The filter unit may include a housing defining an internal volume, the housing having a partition to divide the internal volume into a plurality of portions, wherein each portion is electromagnetically shielded from the other portions by the housing and the partition. Two signal lines may be routed through different portions of the internal volume.

The apparatus may comprise an electromagnetically-shielded conduit extending through a second aperture in a wall of the electromagnetically-shielded enclosure, wherein the conduit is configured to route an optical fibre through the wall. Alternatively or additionally, the apparatus may further include a further filter located outside the filter unit and inside the electromagnetically-shielded enclosure, the further filter being configured to filter noise from at least one of the one or more signal lines. The further filter may comprise a filter circuit having a stopband, wherein the filter circuit is configured such that a scan frequency of a medical imaging device is within the stopband.

The filter unit may be provided independently. A further aspect of the present disclosure provides a filter unit for filtering noise from one or more signal lines of an apparatus for positioning a patient for medical imaging by a medical imaging device, the filter unit comprising one or more filter circuits each configured to filter a respective one of the one or more signal lines, each filter circuit having a respective stopband, wherein each filter circuit is configured such that a scan frequency of the medical imaging device is within the respective stopband.

The filter unit may further include an electromagnetically-shielded housing, and wherein each filter circuit is located within the housing. The housing may define an internal volume and may include a partition to divide the internal volume into a plurality of portions, wherein each portion is electromagnetically shielded from the other portions by the housing and the partition. Two signal lines may be routed through different portions of the internal volume. The filter unit may be configured to be mounted in an aperture in a wall of an electromagnetically-shielded enclosure in which the medical imaging device is located. The filter circuit may be a passive filter including a ladder network of one or more inductors and one or more capacitors.

### BRIEF DESCRIPTION OF THE DRAWINGS

Embodiments will now be described, purely by way of example, with reference to the accompanying drawings, wherein like elements are indicated using like reference signs, and in which:
Figure 1 is a schematic diagram of a conventional MRI system;
Figure 2 is a schematic diagram of a patient positioning apparatus in accordance with the present disclosure;
Figure 3 is perspective view of a housing for a filter unit;
Figure 4 is an exploded view of the housing shown in Figure 3;
Figure 5 is an internal view of the housing shown in Figures 3 and 4;
Figure 6 is a circuit diagram of an example of a filter circuit for use with a filter unit;
Figure 7 shows the frequency response of the filter circuit shown in Figure 6;
Figure 8 is a schematic diagram of a variation of the patient positioning apparatus shown in Figure 2; and
Figure 9 is a partial perspective view of a conduit for routing an optical fibre.

### DETAILED DESCRIPTION

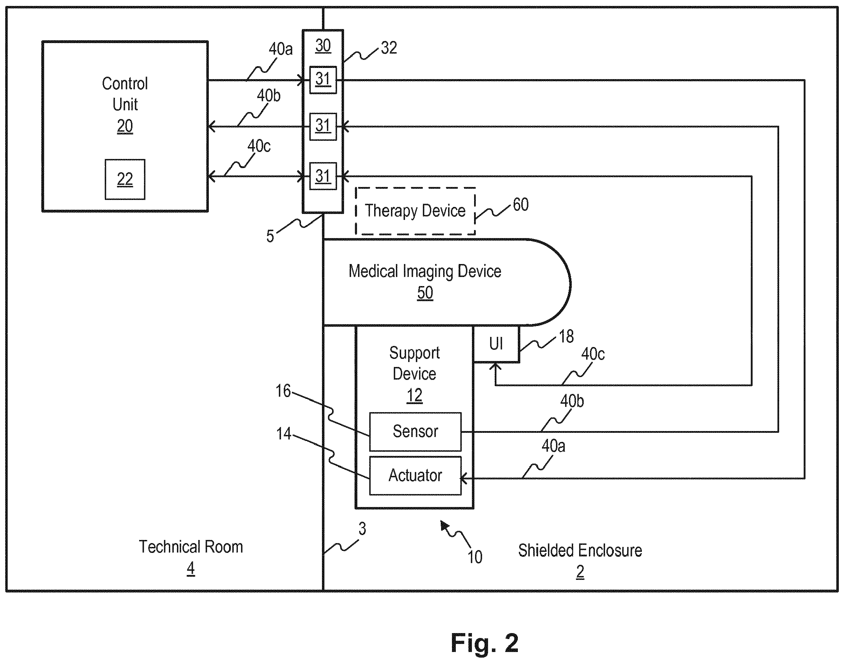

Figure 2 is a schematic diagram of a patient positioning apparatus in accordance with the present disclosure. The patient positioning apparatus comprises a support system 10, a control unit 20 and a filter unit 30. The patient positioning apparatus is configured to position a patient for a medical imaging process performed by a medical imaging device 50. The patient positioning apparatus may optionally be configured to position a patient for a therapeutic process performed by a therapy device 60.

The support system 10 comprises a patient support device 12 and one or more actuators 14. In certain embodiments, the support system 10 may further comprise one or more sensors 16 and a user interface 18. The patient support device 12 is configured to support the body of a patient, or a part of the patient's body, during the medical imaging process. The patient support device 12 is typically in the form of a table, bench or bed upon which the patient can lie during the medical imaging process. The patient support device 12 is movable. The patient support device 12 may have any number of degrees of freedom. For example, the patient support may be capable of translational movement along one, two or three mutually perpendicular axes and/or may be capable of rotation around any axis.

Each actuator 14 is configured to move the patient support device 12. More specifically, each actuator 14 can move the patient support device 12 relative to the medical imaging device 50 and/or the therapy device 60. Each actuator 14 may comprise an electric motor, an electric linear actuator or any other type of actuator capable of moving the patent support device 12. The number of actuators 14 present in the support system 10 depends upon, for example, the number of degrees of freedom of the patient support device 12.

Each sensor 16 is configured to output a signal representing a physical property of the support system 10. The one or more sensors 16 may comprise a sensor configured to output a signal indicative of the position or movement of the patient support device 12. For example, the sensor 16 may be a mechanical switch that is configured to generate a signal when the patient support device 12 is moved to a predetermined location. As another example, if an actuator 14 is a motor, the sensor 16 may be a rotary encoder that measures the angular position of the motor's shaft, and outputs a signal from which the position or movement of the patient support device 12 can be derived. The one or more sensors 16 may additionally or alternatively comprise sensors that are configured to measure the speed, acceleration, deceleration, torque and/or temperature of the actuator 14.

The user interface (UI) 18 is configured to receive a user input indicative of a desired position or a desired movement of the patient support device 12. For example, the user interface 18 may comprise buttons and/or a touch screen that a user can activate in order to cause the patient support device 12 to move to a predetermined position and/or in a specified direction. In response to receiving a user input, the user interface 18 is configured to output a signal indicative of the desired position or desired movement of the patient support device 12.

The control unit 20 is configured to adjust the position of the patient support device 12. To this end, the control unit 20 is communicatively coupled to the patient support system 10 via one or more signal lines 40. More specifically, the control unit 20 can communicate with the one or more actuators 14 via a first signal line 40a, with the one or more sensors 16 via a second signal line 40b, and with the user interface 18 via a third signal line 40c. Each signal line 40 comprises one or more electrical conductors. For example, a signal line 40 may comprise: a cable comprising a single wire; a cable comprising a plurality of wires; or a plurality of cables, each comprising one or more wires. Each signal line 40 may be thus be configured to carry one or more signals.

The control unit 20 may be configured to provide a signal to an actuator 14 via the first signal line 40a. The actuator 14 may be configured to move the patient support device 12 in response to receiving the signal. For example, the control unit 20 may be configured to supply power to the actuator 14 via the first signal line 40a, such that the received power causes the actuator 14 to move the patient support device 12. The control unit 20 may also be configured to stop the supply of power to the actuator 14, so as to cease the movement of the patient support device 12. In a variant of this example, the control unit 20 may pulse width modulate the power supplied to the actuator 14. In another example, the control unit 20 does not supply power to the actuator 14, but instead provides a control signal that causes the actuator 14 to move and/or to stop moving. The control unit 20 may be further configured to receive a signal indicative of the position or movement of the patient support device 12 via the second signal line 40b. The control unit 20 may be further configured to receive a signal indicative of a desired position or a desired movement of the patient support device from the user interface 18 via the third signal line 40c.

The control unit 20 may thus implement a closed loop control system in which: the input is the signal indicative of the desired position or desired movement of the patient support device 12 that is received from the user interface 18 via the third signal line 40c; the output is the signal provided to the one or more actuators 14 via the first signal line 40a; and the feedback is the signal indicative of the position or movement of the patient support device 12 that is received from the one or more sensors 16 via the second signal line 40b.

The control unit 20 may comprise one or more processors 22 that are configured to perform the functions described above. The use of a processor 22 is advantageous because its high processing speed allows the feedback signal in the above-mentioned closed loop system to be sampled and processed at a higher rate than is possible with the conventional control unit 190 shown in Figure 1. Thus, the control unit 20 may achieve more accurate and precise positioning of the patient support device 12 than the conventional control unit 190. Additionally, a processor-based control unit 20 can be implemented using off-the-shelf components, rather than the specially-designed analogue and digital circuits that are used in the conventional control unit 190. For example, the control unit 20 can be implemented using a programmable logic controller (PLC) together with a real-time operating system. Such PLCs are used in automated assembly lines, and are available from suppliers such as Siemens AG, ABB Ltd, Bernecker + Rainer Industrie Elektronik GmbH, Beckhoff Automation GmbH & Co. KG and Rockwell Automation, Inc. A processor-based control unit 20 may also be easier to design, maintain, update and/or reconfigure than the conventional control unit 190.

However, the presence of a processor 22 may cause the control unit 20 to generate a higher level of electromagnetic noise than the conventional control unit 190. The patient positioning apparatus of the present disclosure is configured to avoid such noise from interfering with the medical imaging device 50, as will now be explained.

The patient support system 10 and the medical imaging device 50 are located within an electromagnetically-shielded enclosure 2. The shielded enclosure 2 may be an existing electromagnetically-shielded examination room, such as the examination room 102 described above with reference to Figure 1. However, the electromagnetically-shielded enclosure 2 can be any wholly or partially enclosed space that is configured to attenuate electromagnetic noise that originates outside the shielded enclosure 2 to a sufficient extent that the noise does not interfere with the medical imaging device 50. For example, the electromagnetically-shielded enclosure 2 may comprise a room lined with electrically conductive mesh, foil and/or plates.

The control unit 20 is located outside the electromagnetically-shielded enclosure 2. The electromagnetically-shielded enclosure 2 thus prevents electromagnetic noise generated by the electrical components of the control unit 20 from interfering with the medical imaging device 50. This allows greater freedom in the design of the control unit 20 and, in particular, allows high frequency components (such as the processor 22) to be used without adversely affecting the quality of images produced by the medical imaging device 50. The control unit 20 may be located in a so-called "technical room" 4 that is adjacent an existing electromagnetically-shielded examination room. The technical room 4 typically contains various components of the medical imaging device 50, such as its power supply, that cannot conveniently be located in the examination room.

Locating the control unit 20 outside the electromagnetically-shielded enclosure 2 may give rise to further advantages. For example, the control unit 20 may be inspected or serviced without requiring personnel to be exposed to the high magnetic field that may exist when the medical imaging device 50 is an MRI scanner and/or the radiation that may exist when the therapy device 60 is a radiotherapy device. As another example, there may be more space available in the technical room than in a typical examination room. Hence, locating the control unit 20 in the technical room may facilitate physical access to the control unit 20 and/or may allow better cooling of the control unit 20 and/or may allow the control unit 20 to have larger dimensions than the conventional control unit 190 shown in Figure 1.

The filter unit 30 allows communication between the control unit 20 and the positioning system 10, while also preventing electromagnetic noise generated by the electrical components of the control unit 20 from interfering with the medical imaging device 50. The filter unit 30 comprises a housing 32 and one or more filter circuits 31 located within the housing 32. The housing 32 is electromagnetically shielded.

In some embodiments, the filter unit 30 is located in an aperture 5 in a wall 3 of the electromagnetically-shielded enclosure 2. For example, an aperture may be made in a wall that separates an existing examination room from a technical room, and the housing 32 may be mounted in the aperture. The housing 32 may be electrically connected to an electrically conductive material that provides electromagnetic shielding to the shielded enclosure 2, such that the housing 32 and the electromagnetic shielding are at the same electric potential. This helps to avoid the effectiveness of the electromagnetic shielding of the shielded enclosure 2 being compromised by the presence of the aperture 5 and filter unit 30. The dimensions of the aperture 5 may be substantially the same as the external dimensions of the housing 32, such that there is little or no gap between the aperture 5 and the housing 32. For example, there may be an interference fit or a transition fit between the aperture 5 and the housing 32. This also helps to avoid the effectiveness of the electromagnetic shielding of the shielded enclosure 2 being compromised by the presence of the aperture 5 and filter unit 30.

The filter circuits 31 are configured to filter noise from the signal lines 40. More specifically, the filter circuits 31 are configured to prevent noise being transmitted into the shielded enclosure 2 via the signal lines 40. In particular, the filter circuits 31 are configured to attenuate noise generated by the electrical components of the control unit 20, and thus prevent that noise from being transmitted into the shielded enclosure 2 via the signal lines 40. However, the filter circuits 31 can also be configured to attenuate other sources of noise that originate outside the shielded enclosure 2 from being transmitted into the shielded enclosure 2 via the signal lines 40. Each of the signal lines 40a, 40b, 40c may be connected to a respective filter circuit 31. The filter characteristics (such as cut-off frequency and roll-off) of each filter circuit 31 are selected to prevent noise originating outside the shielded enclosure 2 from interfering with the medical imaging device 50. The filter characteristics of the filter circuits 31 may depend upon the operating parameters of a particular imaging device 50. For example, if the imaging device 50 is an MRI scanner, each filter circuit 31 is designed such that the resonance frequency of the MRI scanner is within the stopband of the filter. Other types of imaging device 50 may have other frequencies at which they are sensitive to interference from electromagnetic noise. Hence, the filter characteristics of the filter circuit 31 may be optimised for a particular imaging device 50. In general, the filter circuit 31 is designed such that the scan frequency of the imaging device 50 is within the stopband of the filter. The filter circuit 31 may be an active filter or a passive filter. The filter circuit 31 may be a low-pass filter, a band-pass filter or a band-stop filter. The term "stopband" as used herein generally refers to a frequency, or a range of frequencies, that are attenuated by the filter circuit 31. As is known to those skilled in the art, the stopband may be defined by the cut-off frequency (or frequencies) of the filter circuit 31. For example, in the case of a low-pass filter, the stopband may be regarded as all frequencies above the cut-off frequency. The cut-off frequency may be defined as the frequency at which the power output of the filter is half the power output in the passband of the filter.

The medical imaging device 50 can be any device configured to produce a two-dimensional or three-dimensional medical image. The medical imaging device may be, for example, an MRI scanner, an X-ray machine, a computed tomography (CT) scanner, a positron emission tomography (PET) scanner or an ultrasound scanner. The low level of electromagnetic noise produced by the patient positioning apparatus disclosed herein may be particularly advantageous when the medical imaging device 50 is an MRI scanner, since MRI scanners are particularly sensitive to interference. The medical imaging device 50 operates at a scan frequency. The term "scan frequency" may refer to a frequency, or a range of frequencies, that is determined by the physical phenomenon that the medical imaging device 50 relies upon to produce medical images. For example, the term "scan frequency" may be the resonance frequency of an MRI scanner or the ultrasound frequency of an ultrasound scanner. More generally, however, the term "scan frequency" may include any frequency, or any range of frequencies, at which the medical imaging device 50 is sensitive to interference from electromagnetic noise, wherein such interference causes degradation of the medical images generated by the medical imaging device 50.

The therapy device 60 can be any device that can perform any type of therapy, treatment or surgery upon a patient. For example, the therapy device 60 may be a radiotherapy device, such a linear accelerator, which is configured to kill cancerous cells with a beam of ionising radiation. In this example, the medical imaging device 50 and radiotherapy device can be used together to perform image guided radiotherapy (IGRT). In IGRT, a medical image produced by the medical imaging device 50 is used to direct the beam produced by the radiotherapy device towards a region of the patient that is to be treated, such as a tumour. In this manner, IGRT can target cancerous cells more precisely and reduce the exposure of non-cancerous cells to radiation. The present disclosure can allow more accurate and precise control systems to be used for controlling the position of the patient support device 12, without increasing the level of electromagnetic noise in the vicinity of the medical imaging device 50. The improved accuracy and precision in positioning the patient support device 12 can help to ensure that the region of the patient that is to be treated is positioned accurately and precisely with respect to the beam. In this manner, the accuracy of IGRT can be further improved.

In one example of an IGRT system in accordance with the present disclosure, the medical imaging device 50 is an MRI scanner, and the therapy device 60 is a linear accelerator. The combination of an MRI scanner and a linear accelerator, known as an "MR-LINAC", results in particularly precise treatment of cancerous cells. As noted above, the low level of electromagnetic noise produced by the patient positioning apparatus disclosed herein may be particularly advantageous when the medical imaging device 50 is an MRI scanner, since MRI scanners are particularly sensitive to interference.

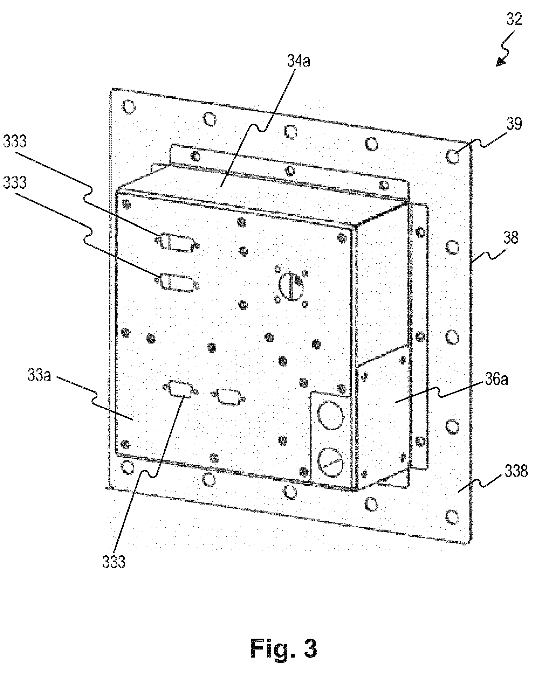

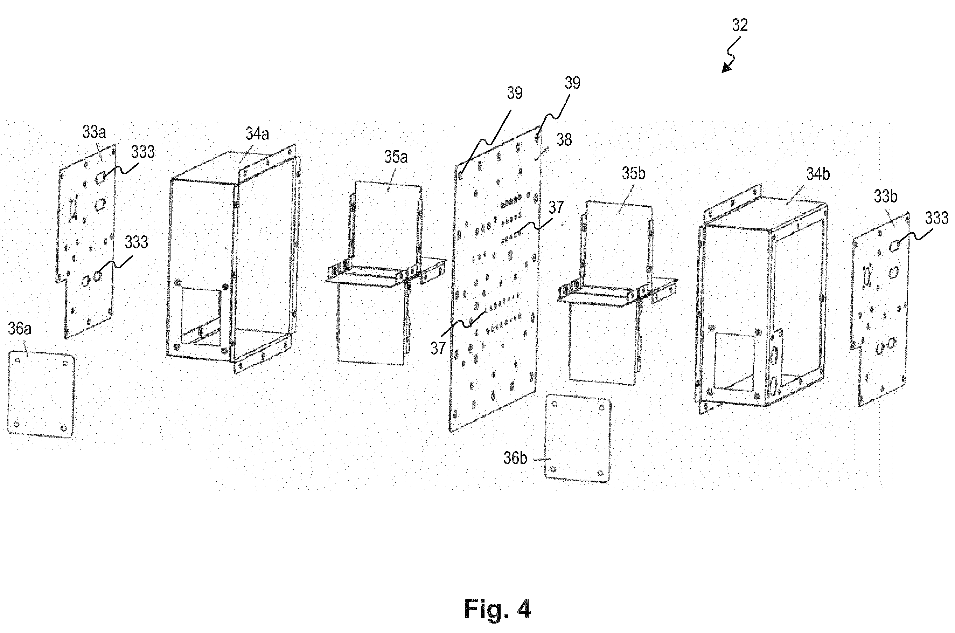

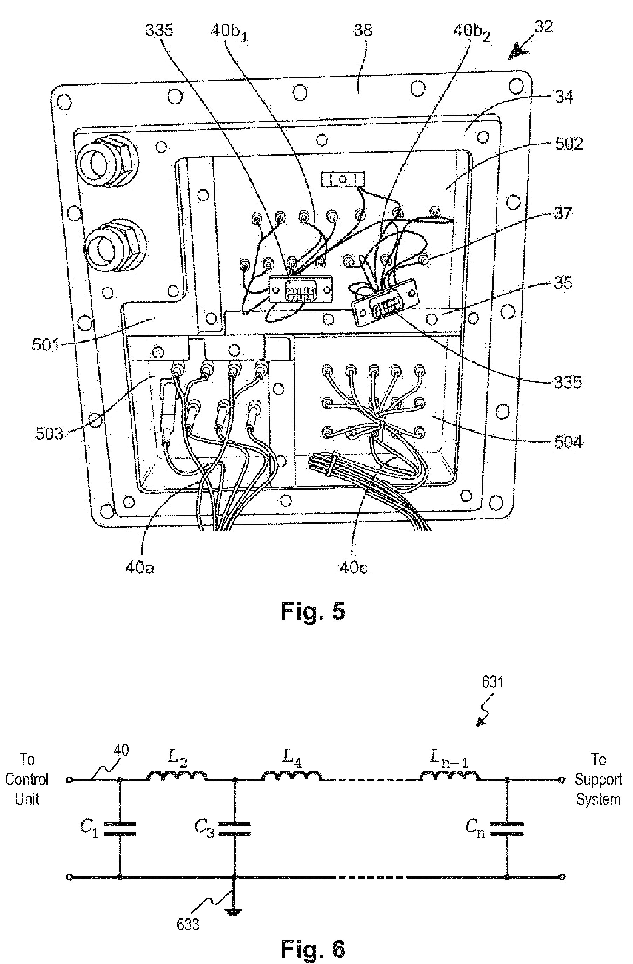

An example of a housing 32 for a filter unit 30 will now be described with reference to Figures 3, 4 and 5, which respectively show a perspective view, an exploded view and an internal view of the housing 32. The housing 32 comprises two end plates 33a, 33b, two box sections 34a, 34b, two partitions 35a, 35b, two side plates 36a, 36b, and one centre plate 38. The housing 32 is symmetric about the centre plate 38, such that the components 33a, 34a, 35a, 36a disposed on one side of the centre plate 38 are mirror images of the components 33b, 34b, 35b, 36b disposed on the other side of the centre plate 38. Each box section 34 comprises several openings, which are closed by a respective end plate 33, side plate 36 and centre plate 38 when the housing 32 is assembled. Thus, the housing 32 has an internal volume defined by the internal surfaces of the box portions 34, the end plates 33 and the side plates 36. Each partition 35 is dimensioned to fit inside a respective box section 34. The partitions 35a, 35b and the centre plate 38 divide the internal volume of the housing 32 into a plurality of portions. For example, the internal volume of the housing 32 shown in Figures 3 to 5 is divided into eight portions. Four portions 501, 502, 503, 504 are visible in Figure 5, and there are a further four portions (not visible in Figure 5) at corresponding positions on the opposite side of the centre plate 38. The region of the centre plate 38 that is within the internal volume of the housing 32 may comprise one or more through holes 37. Each through hole 37 allows one or more signal lines 40a, 40b, 40c to pass through the centre plate 38. Each end plate 33a, 33b may comprise one or more holes 333 to which an electrical connector 335 (shown in Figure 5) may be mounted. An electrical connector 335 on end plate 33a can be electrically connected to an electrical connector 335 on end plate 33b, via a filter circuit 31, by a conductor that passes through the internal volume of the housing 32. The electrical connectors 335 allow the filter unit 30 to be detachably connected to the support system 10 and the control unit 20.

The housing 32 is constructed from an electrically conductive material. The conductive material, and the thickness thereof, are selected to ensure that the housing 32 provides effective electromagnetic shielding against noise originating outside the shielded enclosure 2. When the housing 32 is assembled, its components 33, 34, 35, 36, 38 are fixed to each other by electrically conducting screws (not shown in Figures 3 to 5), such that all of the components are at the same electrical potential. The exterior of the housing 32 is thus electromagnetically shielded from the internal volume of the housing 32. Furthermore, each portion 501, 502, 503, 504 of the internal volume of the housing 32 is electromagnetically shielded from the other portions 501, 502, 503,504.

Different signal lines 40a, 40b, 40c may be routed through different portions 501, 502, 503, 504 of the housing 32. For example, signal lines carrying signals whose voltages and/or frequencies differ significantly may be routed through different portions 501, 502, 503, 504. Since each portion 501, 502, 503, 504 is electromagnetically shielded from the other portions 501, 502, 503, 504, routing the signal lines 40a, 40b, 40c in this manner can reduce crosstalk between signal lines. Thus, the electromagnetic shielding between different portions 501, 502, 503, 504 of the housing 32 can help to avoid signal degradation that might otherwise occur when the signal lines 40a, 40b, 40c are placed in close proximity to each other. Reducing crosstalk can also improve the effectiveness of the filter unit 30 in preventing noise generated in the control unit 20 from interfering with the medical imaging device 50.

Figure 5 shows an example of how signal lines 40 can be routed through different portions 502, 503, 504 of the housing 32 to reduce crosstalk. Portion 502 comprises sensor signal lines, 40b₁ and 40b₂. Signal line 40b₁ carries a signal from a cable-pull sensor, which has a nominal amplitude of 2.5 V and a nominal frequency of 125 kHz. Signal line 40b₂ carries a signal from a rotary encoder sensor, which has a nominal amplitude of 2.5 V and a nominal frequency of 6 MHz. Since the amplitudes of the signals carried by signal lines 40b₁ and 40b₂ are relatively small, there is a low risk of crosstalk between subharmonics from signal lines 40b₁ and 40b₂ causing image degradation at the scan frequency of the medical imaging device 50. Hence, signal lines 40b₁ and 40b₂ can be routed through the same portion 502 of the housing. Portion 503 comprises an actuator signal line 40a. Signal line 40a carries a pulse width modulated power signal, which has a nominal amplitude of 315 V and a nominal frequency of 5 kHz. Portion 504 comprises user interface signal lines 40c, which carry signals with a low voltage and very low frequency. Routing the user interface signal lines 40c through a dedicated portion 504 of the housing 32 can prevent the user interface signals being degraded by interference from signal lines 40a, 40b₁ and 40b₂ which carry higher voltage and higher frequency signals.

It will be appreciated that the housing 32 may comprise any number of portions. The number of portions may be selected depending upon the number of signal lines 40 and the characteristics of the signals carried by those signal lines 40. The signal lines 40 may be routed through the housing 32 in a different manner from that described above. The particular manner in which the signal lines 40 are routed through the housing 32 can be selected based upon the characteristics of the signals carried by the signal lines 40 and the signals' sensitivity to noise.

As shown in Figure 3, the area of the centre plate 38 may be greater than the cross-sectional area of the box sections 34. Thus, the periphery of the centre plate 38 (i.e. the area of the centre plate 38 that protrudes from the internal volume of the housing 32) forms a flange 338. The flange 338 comprises one or more through holes 39.

To install the filter unit 30, an aperture 5 is made in the wall 3 of the electromagnetically-shielded enclosure 2. The dimensions of the aperture 5 may be the same as the external dimensions of a box section 34. The box section 34 is pushed through the aperture 5 until the flange 338 abuts the wall 3. The housing 32 can then be secured to the wall 3 by fasteners that pass through the through holes 39 and into the wall 3. Furthermore, an electrically conductive fastener (such as a metal screw) can be used both to secure the housing 32 and to electrically connect the housing 32 to the electrically conductive material that provides electromagnetic shielding to the shielded enclosure 2. By installing the filter unit 30 in this manner, the centre plate 38 substantially closes the aperture 5 in the wall 3 of the electromagnetically-shielded enclosure 2. Hence, the effectiveness of the electromagnetic shielding of the shielded enclosure 2 is not compromised by the presence of the aperture 5 and filter unit 30.

An example of a filter circuit 31 will now be described with reference to Figure 6. Figure 6 is a circuit diagram of a filter circuit 631, which comprises a ladder network of one or more capacitors, C₁, C₃ ... Cₙ, connected in parallel with one or more inductors, L₂, L₄ ... Lₙ₋₁. The ground 633 of the filter circuit 631 may be connected to the housing 32 of the filter unit 30. The number of capacitors and inductors, and their respective capacitance and inductance values, are selected in accordance with known filter design techniques to prevent noise originating outside the electromagnetically-shielded enclosure 2 (and particularly noise generated by the control unit 20) from causing interference to the medical imaging device 50. For example, if the imaging device 50 is an MRI scanner with a scan frequency of 64 MHz, the capacitance and inductance values can be selected to implement a low-pass filter with a cut-off frequency below 64 MHz.

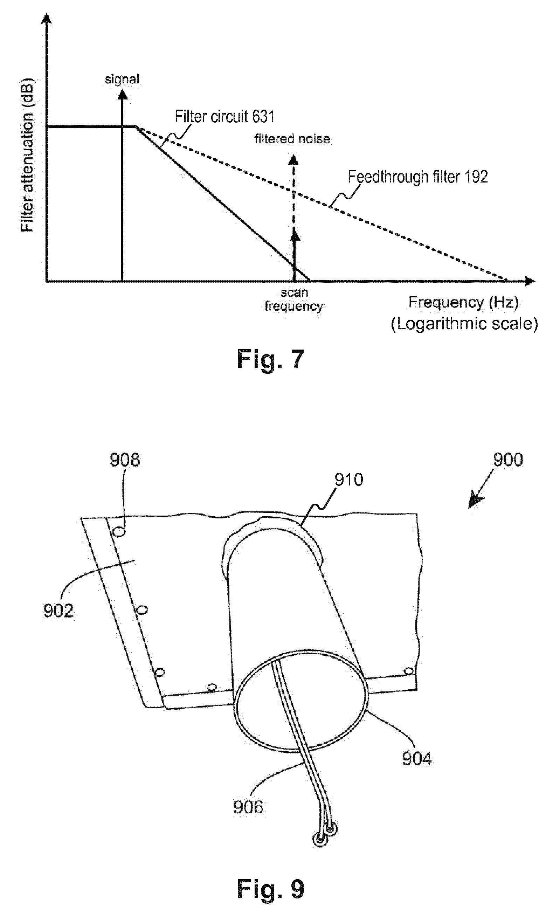

The filter circuit 631 is advantageous in that does not comprise any active components and, therefore, is inexpensive, compact and simple to implement. Further, the filter circuit 631 is particularly compact when implemented using surface mounted components, thus allowing a relatively small housing 32 to be used. Another advantage of the filter circuit 631 is that it can be designed to provide greater attenuation of noise at the scan frequency of the medical imaging device 50 than is possible using the feedthrough filters 192 that are present in the conventional MRI system shown in Figure 1. In particular, the filter circuit 631 can be designed as a second (or higher) order filter, whereas the feedthrough filters 192 are typically first order filters. Figure 7 illustrates that the filter circuit 631 can be designed to have a steeper roll-off than can be achieved with a feedthrough filter 192, resulting in less noise at the scan frequency of the imaging device 50.

The inductance and capacitance values of the filter circuit 631 can be selected such that the filter is symmetrical. When the filter circuit 631 is symmetrical, the frequency response of the filter to a signal applied to the left of the circuit is identical to that for a signal applied to the right of the circuit. Consequently, the time delay introduced by the filter circuit 631 for a signal applied to the left of the circuit is equal to that for a signal applied to the right of the circuit. The filter circuit 631 can thus be used to filter bidirectional signal communication lines. The filter circuit 631 can be made symmetrical by setting C₁ equal to Cₙ and by setting L₂ equal to Lₙ₋₁.

It will be appreciated that the filter circuit 631 is merely an example, and that other suitable filter circuits may fall within the scope of the present disclosure.

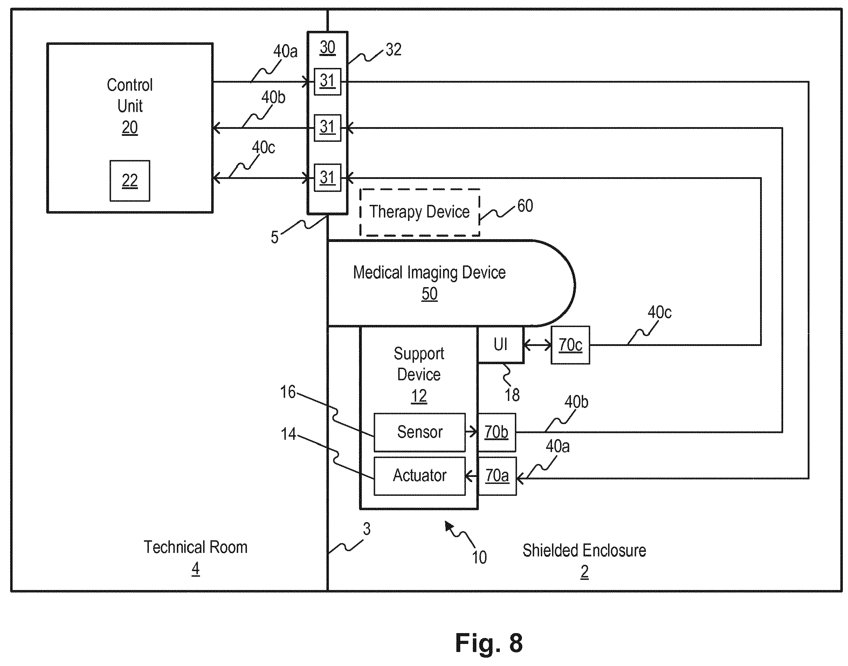

The control unit 20 can be prevented from interfering with the medical imaging device by locating the control unit 20 outside the shielded enclosure 2 and by the filter unit 30 described herein. However, other components of the patient positioning apparatus may also interfere with the medical imaging device 50. In particular, the actuator 14, the sensor 16 and/or the user interface 18 may generate electromagnetic noise that can degrade the quality of the medical images generated by the medical imaging device 50. If any of these components cause interference, further filters 70 may be added to the patient positioning apparatus as shown in Figure 8.

The patient positioning apparatus shown in Figure 8 is substantially the same as that shown in Figure 2, but further filters 70a, 70b, 70c have been added to signal lines 40a, 40b, 40c respectively. The further filters 70a, 70b, 70c are designed such that the scan frequency of the medical imaging device 50 is within their stopbands. For example, the further filters 70 may comprise filter circuits with the same design as the filter circuit 631 shown in Figure 6. The further filters 70a, 70b, 70c may be placed as close as possible to the actuator 14, sensor 16 and user interface 18 respectively, so as to prevent noise generated by the actuator 14, sensor 16 and user interface 18 being transmitted via the signal lines 40a, 40b, 40c. Any or all of the further filters 70a, 70b, 70c may be omitted if the actuator 14, sensor 16 and user interface 18 do not interfere with the medical imaging device 50.

A consequence of locating the control unit 20 outside the electromagnetically-shielded enclosure 2 is that signal lines pass through the wall 3 of the shielded enclosure 2. The filter unit 30 allows signal lines 40a, 40b, 40c to pass through the wall 3 without compromising the effectiveness of the electromagnetic shielding of the shielded enclosure 2. However, it may not be appropriate to route all signal lines through the filter unit 20. For example, the frequency of a signal may be so close to the scan frequency of the medical imaging device 50 that it cannot be adequately filtered to prevent interference with the medical imaging device 50. In this case, interference may be avoided by converting the signal to an optical signal and carrying the optical signal via an optical fibre, rather than by an electrical conductor.

Figure 9 shows a conduit 900 for allowing an optical fibre to pass through the wall 3 of the electromagnetically-shielded enclosure 2 without compromising the effectiveness of the electromagnetic shielding of the shielded enclosure 2. The conduit 900 can also be used to route electrical wires and cables through the wall 3. The conduit 900 comprises a flange 902 and a hollow tube 904. The tube 904 extends through the flange 902. Whilst Figure 9 shows a tube 904 with a circular cross-section, it will be appreciated that its cross-section need not be circular. The flange comprises one or more through holes 908. The flange 902 and the tube 904 are each formed from an electrically conductive material. The flange 902 may be physically secured and electrically connected to the tube 904 by a welded or soldered joint 910.

To install the conduit 900, an aperture (not shown in Figure 2) is made in the wall 3 of the electromagnetically-shielded enclosure 2. The dimensions of the aperture may be the same as the external dimensions of the tube 904. The tube 904 is pushed through the aperture until the flange 902 abuts the wall 3. The conduit 900 can then be secured to the wall 3 by fasteners that pass through the through holes 908 and into the wall 3. Furthermore, an electrically conductive fastener (such as a metal screw) can be used both to secure the conduit 900 and to electrically connect the conduit 900 to the electrically conductive material that provides electromagnetic shielding to the shielded enclosure 2. An optical fibre 906 or an electrical cable can be passed through the hollow interior volume of the tube 904. By installing the conduit in this manner, the flange 902 and tube 904 substantially close the aperture in the wall 3 of the electromagnetically-shielded enclosure 2. Hence, the effectiveness of the electromagnetic shielding of the shielded enclosure 2 is not compromised by the presence of the aperture and conduit 900.

It will be understood that the invention has been described above purely by way of example, and that modifications of detail can be made within the scope of the invention.

## Claims

1. An apparatus for positioning a patient for medical imaging, the apparatus comprising:
a support system comprising a patient support device and an actuator configured to move the patient support device, wherein the support system is located inside an electromagnetically-shielded enclosure;
a control unit configured to adjust the position of the patient support device by communicating with the support system via one or more signal lines, wherein the control unit is located outside the electromagnetically-shielded enclosure; and
a filter unit configured to filter noise from the signal lines.

2. An apparatus in accordance with claim 1, wherein the filter unit is located in a first aperture in a wall of the electromagnetically-shielded enclosure.

3. An apparatus in accordance with claim 1 or claim 2, wherein the filter unit further includes an electrically-conductive housing, the housing being electrically connected to the wall.

4. An apparatus in accordance with any of the preceding claims, wherein the filter unit further includes a filter circuit having a stopband, wherein the filter circuit is configured such that a scan frequency of a medical imaging device is within the stopband.

5. An apparatus in accordance with claim 4, wherein the filter circuit is a passive filter including a ladder network of one or more inductors and one or more capacitors.

6. An apparatus in accordance with any of the preceding claims, wherein:
the control unit is further configured to provide a signal to the actuator via a first signal line of the one or more signal lines, wherein the actuator is further configured to move the patient support device in response to receiving the signal.

7. An apparatus in accordance with any of the preceding claims, wherein the support system further includes a sensor configured to output a signal indicative of a position or movement of the patient support device, and wherein the control unit is further configured to receive the signal indicative of the position or movement of the patient support device via a second signal line of the one or more signal lines.

8. An apparatus in accordance with any of the preceding claims, wherein the support system further includes a user interface configured to:
receive a user input indicative of a desired position or a desired movement of the patient support device;
output a signal indicative of the desired position or desired movement of the patient support device to the control unit via a third signal line of the one or more signal lines.

9. An apparatus in accordance with any of the preceding claims, wherein the filter unit further includes a housing defining an internal volume, the housing having a partition to divide the internal volume into a plurality of portions, wherein each portion is electromagnetically shielded from the other portions by the housing and the partition.

10. An apparatus in accordance with claim 9, wherein two signal lines are routed through different portions of the internal volume.

11. An apparatus in accordance with any of the preceding claims, further including an electromagnetically-shielded conduit extending through a second aperture in a wall of the electromagnetically-shielded enclosure, wherein the conduit is configured to route an optical fibre through the wall.

12. An apparatus in accordance with any of the preceding claims, further including a further filter located outside the filter unit and inside the electromagnetically-shielded enclosure, the further filter being configured to filter noise from at least one of the one or more signal lines.

13. An apparatus in accordance with claim 12, wherein the further filter further includes a filter circuit having a stopband, wherein the filter circuit is configured such that a scan frequency of a medical imaging device is within the stopband.

14. A filter unit for filtering noise from one or more signal lines of an apparatus for positioning a patient for medical imaging by a medical imaging device, the filter unit comprising:
one or more filter circuits each configured to filter a respective one of the one or more signal lines, each of the one or more filter circuits having a respective stopband, wherein each of the one or more filter circuits is configured such that a scan frequency of the medical imaging device is within the respective stopband.

15. A filter unit in accordance with claim 14, wherein:
the filter unit further includes an electromagnetically-shielded housing, and wherein each filter circuit is located within the housing, and, optionally,
the housing defines an internal volume and includes a partition to divide the internal volume into a plurality of portions, wherein each portion is electromagnetically shielded from the other portions by the housing and the partition, and, optionally,
two signal lines are routed through different portions of the internal volume; and/or
the filter unit is configured to be mounted in an aperture in a wall of an electromagnetically-shielded enclosure in which the medical imaging device is located; and/or
a filter circuit is a passive filter including a ladder network of one or more inductors and one or more capacitors.
